# EUROPÄISCHE PATENTSCHRIFT

(11) **EP 2 670 453 B1**
(45) Veröffentlichungstag und Bekanntmachung des Hinweises auf die Patenterteilung: **13.12.2017**
(21) Anmeldenummer: 12704668.8
(22) Anmeldetag: 24.01.2012
(51) Int. Cl.: A61M 1/16, A61M 1/34

(54) **VERFAHREN UND VORRICHTUNG ZUR STEUERUNG EINER EXTRAKORPORALEN BLUTBEHANDLUNGSVORRICHTUNG**
METHOD AND DEVICE FOR CONTROLLING AN EXTRACORPOREAL BLOOD TREATMENT DEVICE
PROCÉDÉ ET DISPOSITIF DE COMMANDE D'UN DISPOSITIF DE TRAITEMENT EXTRACORPOREL DU SANG

(30) Priorität: 01.02.2011 DE 102011010067; 01.02.2011 US 201161438275 P
(43) Veröffentlichungstag der Anmeldung: 11.12.2013
(73) Patentinhaber: Fresenius Medical Care Deutschland GmbH, 61352 Bad Homburg v.d.H. (DE)
(72) Erfinder: BREUEL, Lars, 99096 Erfurt (DE); LINDNER, Thomas, 91301 Forchheim/Ofr. (DE); BEDEN, Josef, 55252 Mainz-Kastel (DE); HERKLOTZ, Martin, 63150 Heusenstamm (DE); VERCH, Georg, 65191 Wiesbaden (DE)
(74) Vertreter: Oppermann, Frank
(86) Internationale Anmeldenummer: PCT/EP2012/000292
(87) Internationale Veröffentlichungsnummer: WO 2012/104026

(56) Entgegenhaltungen:
- EP-A1- 1 543 853
- EP-A1- 2 019 296
- WO-A1-98/50091
- US-A- 5 776 345

## Beschreibung

Die Erfindung betrifft ein Verfahren und eine Vorrichtung zur Steuerung einer extrakorporalen Blutbehandlungsvorrichtung, die insbesondere als Hämodialysevorrichtung, Hämofiltrationsvorrichtung oder Hämodiafiltrationsvorrichtung betrieben werden kann. Darüber hinaus betrifft die Erfindung eine extrakorporale Blutbehandlungsvorrichtung mit einer derartigen Steuerungsvorrichtung.

Bei einer Hämodialysebehandlung strömt das zu behandelnde Blut durch die erste Kammer eines durch eine semipermeable Membran in die erste und eine zweite Kammer unterteilten Dialysators, während Dialysierflüssigkeit durch die zweite Kammer des Dialysators strömt. Die Hämodialysebehandlung erfordert die Bilanzierung von frischer gegen verbrauchte Dialysierflüssigkeit, um die dem Patienten zugeführte oder entzogene Menge an Flüssigkeit kontrollieren zu können. An die Bilanzierung der Flüssigkeiten werden bei der Hämodialyse hohe Anforderungen gestellt.

In der akuten Dialyse für den Einsatz auf Intensivstationen finden extrakorporale Blutbehandlungsvorrichtungen Verwendung, bei denen die für die Blutbehandlung erforderlichen Flüssigkeiten in Behältnissen, insbesondere Beuteln, bereitgestellt werden. Bei den bekannten Hämodiafiltrationsvorrichtungen für die akute Dialyse werden Dialysierflüssigkeit und Substituat in Beuteln bereitgestellt, während Filtrat in einem Beutel gesammelt wird. Zur Förderung der Flüssigkeiten werden peristaltische Pumpen eingesetzt, an deren Fördergenauigkeit hohe Anforderungen gestellt werden, um die Flussrate von Dialysierflüssigkeit, Substituat und Filtrat genau einstellen zu können.

Die Bilanzierung der Flüssigkeiten erfolgt bei den Blutbehandlungsvorrichtungen, die zum Einsatz auf Intensivstationen bestimmt sind, dadurch, dass das Gewicht der mit den Flüssigkeiten befüllten Beutel überwacht wird. Hierfür verfügen die bekannten Blutbehandlungsvorrichtungen über Waagen. Für die Blutbehandlung werden die Flussraten für Dialysierflüssigkeit (Dialysat), Substituat und Filtrat vorgegeben, die mit den peristaltischen Pumpen eingestellt werden. Da in der Praxis aber die tatsächlichen Förderraten der peristaltischen Pumpen, insbesondere Rollenpumpen, von den bei der Ansteuerung angenommenen Soll-Förderraten abweichen, kann es zu einer fehlerhaften Bilanzierung kommen. Die exakte Bilanzierung wird dadurch erreicht, dass die Förderrate einer oder mehrerer Pumpen derart verändert wird, dass die Differenz zwischen der Gewichtsabnahme pro Zeiteinheit des Dialysat-Beutels und des Substituat-Beutels und der Gewichtszunahme pro Zeiteinheit des Filtrat-Beutels einem vorgegebenen Wert entspricht. Wenn dem Patienten keine Flüssigkeit entzogen werden soll, werden die Pumpen derart geregelt, dass die Summe der Gewichtsabnahme von Dialysat und Substituat exakt der Gewichtszunahme von Filtrat entspricht. Andernfalls wird dem Patienten eine bestimmte Menge an Flüssigkeit zugeführt oder entzogen (Ultrafiltration).

Bei der Bilanzierung muss Berücksichtigung finden, wenn dem Patienten zur Antikoagulanz weitere Flüssigkeiten, beispielsweise Heparin oder Zitrat und Kalzium verabreicht werden. Die Verabreichung weiterer Flüssigkeiten kann durch eine vom Bediener vorgenommene manuelle Einstellung oder automatisch bei dem Filtratfluss berücksichtigt werden. In der folgenden Beschreibung wird aus Vereinfachungsgründen aber darauf nicht näher eingegangen.

Für die Bilanzierung ist grundsätzlich nur die Messung der Summe des Gewichts des Dialysat- und Substituat-Beutels einerseits und des Gewichts des Filtrat-Beutels andererseits erforderlich. Daher sind Blutbehandlungsvorrichtungen bekannt, die nur über zwei Waagen verfügen. Es sind aber auch Blutbehandlungsvorrichtungen mit drei Waagen bekannt, bei denen Dialysat- und Substituat-Beutel mit getrennten Waagen gewogen werden, so dass das Gewicht jedes einzelnen Beutels bestimmt werden kann.

Eine extrakorporale Blutbehandlungsvorrichtung, die über eine Bilanziereinrichtung verfügt, die über eine Dialysat-Pumpe zum Fördern von Dialysat aus einem Dialysat-Behälter in die Dialysierflüssigkeitskammer und eine Filtrat-Pumpe zum Fördern von Filtrat aus der Dialysierflüssigkeitskammer in einen Filtrat-Behälter aufweist, ist aus der EP 2 019 296 A1 bekannt. Mit einer Substituat-Pumpe wird dem extrakorporalen Blutkreislauf Substituat aus einem Substituat-Behälter zugeführt. Darüber hinaus weist die Bilanziereinrichtung eine Waage zum Wiegen des Dialysat-Behälters und des Substituat-Behälters und eine Waage zum Wiegen des Filtrat-Behälters auf. Zur Bilanzierung wird die Drehzahl der Pumpen mit einer Regeleinheit derart geregelt, dass die Differenz zwischen der Summe der Gewichte des Dialysat- und Substituat-Behälters und des Filtrat-Behälters innerhalb eines vorgegebenen Bereichs liegt. Wenn dem Patienten keine Flüssigkeit entzogen werden soll, können die Pumpen derart geregelt werden, dass die Summe der Gewichtsabnahme von Dialysat und Substituat exakt der Gewichtszunahme von Filtrat entspricht. Andernfalls wird dem Patienten Flüssigkeit (Ultrafiltrat) entzogen oder zugeführt.

Die Regeleinheit der bekannten Blutbehandlungsvorrichtungen sehen einen bestimmten Regelbereich für die Flussraten der Pumpen vor, der in der Praxis beispielsweise +/- 20 % des eingestellten Soll-Flusses betragen kann. Wenn eine Fehlbilanzierung vorliegen sollte, kann durch Veränderung der Flussrate einer oder mehrerer Pumpen innerhalb des Regelbereichs die Fehlbilanzierung ausgeglichen werden. Beispielsweise kann in dem Fall, dass die tatsächliche Förderrate der Dialysatpumpe geringer als die bei der Ansteuerung angenommene Soll-Förderrate ist, die Förderrate der Dialysatpumpe entsprechend erhöht werden. Zum Ausgleich der fehlerhaften Bilanzierung muss die erforderliche Erhöhung der Förderrate der Dialysatpumpe aber innerhalb des Regelbereichs, d.h. beispielsweise innerhalb des Regelbereichs von +/- 20 % der Soll-Flussrate liegen, um die Fehlbilanz ausgleichen zu können. Der Regelzyklus beträgt beispielsweise 1 Sekunde.

Bei den bekannten Waagen zum Wiegen der Beutel handelt es sich um Präzisionswaagen, die regelmäßig überprüft werden müssen. Daher sehen bekannte Blutbehandlungsvorrichtungen zyklische Tests der Waagen vor, die automatisch durchgeführt werden. Mit den zyklischen Waagentests soll nicht nur eine fehlerhafte Bilanzierung aufgrund von Fehlern der Waagen, sondern auch eine Fehlbilanz aufgrund von Undichtigkeiten des Schlauchsystems vermieden werden, die sich bei den Waagentests durch eine unerlaubte Drift der Messwerte zeigen.

Für einen zyklischen Test der Waagen müssen die Pumpen angehalten werden, um die Waagen überprüfen zu können. Daher wird angestrebt, die Anzahl der zyklischen Tests möglichst gering zu halten. Der zeitliche Abstand zwischen aufeinanderfolgenden Waagentests muss dabei so gewählt werden, dass die in der Zwischenzeit unerkannt bleibende Fehlbilanz nicht größer als ein bestimmter Wert, beispielsweise 500 ml, sein kann. Um in der Zwischenzeit unerkannt zu bleiben, muss ein Waagenfehler oder eine Undichtigkeit des Schlauchsystems so gering sein, dass der Waagenfehler oder die Undichtigkeit mit dem erlaubten Regelbereich von beispielsweise +/- 20 % des eingestellten Soll-Flusses ausgeglichen werden kann, so dass es nicht zu einer Bilanzabweichung kommen kann. Folglich ist das Zeitintervall zwischen den Waagentests von der Flussrate und dem erlaubten Regelbereich abhängig. Damit werden auch die Zeitintervalle zwischen den zyklischen Waagentests festgelegt, um zu vermeiden, dass der Grenzwert für die Fehlbilanz zwischen zwei zyklischen Waagentests überschritten wird. Je geringer also der erlaubte Regelbereich ist, umso größer kann das Intervall zwischen den zyklischen Waagentests gewählt werden.

Der Erfindung liegt die Aufgabe zugrunde, ein Verfahren zur Steuerung einer extrakorporalen Blutbehandlungsvorrichtung anzugeben, das eine exakte Bilanzierung der Flüssigkeiten und eine Verlängerung der Intervalle für die Tests der Waagen insbesondere dann erlaubt, wenn für die Bilanzierung der Flüssigkeiten nur die Summe des Gewichts von zwei Flüssigkeiten gemessen wird. Darüber hinaus liegt der Erfindung die Aufgabe zugrunde, eine Vorrichtung zur Steuerung einer extrakorporalen Blutbehandlungsvorrichtung bereit zu stellen, die eine genaue Bilanzierung bei verlängerten Intervallen für die Waagentests ermöglicht. Eine Aufgabe der Erfindung ist auch eine extrakorporale Blutbehandlungsvorrichtung mit einer derartigen Steuerungsvorrichtung bereit zu stellen.

Die Lösung dieser Aufgaben erfolgt erfindungsgemäß mit den Merkmalen der Patentansprüche 1 und 10. Vorteilhafte Ausführungsformen der Erfindung sind Gegenstand der Unteransprüche.

Das Grundprinzip des erfindungsgemäßen Verfahrens und der erfindungsgemäßen Vorrichtung liegt in der Kombination der auf den Gewichten der Flüssigkeits-Behältnisse basierenden Bilanzierung und der Ermittlung der genauen Förderraten der zur Förderung der Flüssigkeiten dienenden Pumpen. Die genaue Kenntnis der Förderraten erlaubt einen kleineren Regelbereich der zur Bilanzierung verwendeten Pumpen und ermöglicht dadurch, Bilanzabweichungen aufgrund von Fehlern der Waagen, Undichtigkeiten im System etc. früher zu erkennen. Ein weiterer Vorteil liegt in der Erhöhung der Behandlungseffektivität durch die Verlängerung der Intervalle für die Tests der Waagen. Die Vorteile eines kleineren Regelbereichs kommen insbesondere dann zum Tragen, wenn nur die Summe des Gewichts von zwei mit Flüssigkeit befüllten Behältnissen ausgewertet wird, beispielsweise nur die Summe des Gewichts von Dialysat- und Substituatbeutel gemessen wird. Dann besteht bei einem zu großen Regelbereich die Gefahr, dass ein zu großer Anteil der beispielsweise von der Substituatpumpe nicht geförderten Menge an Substituat von der Dialysatpumpe übernommen wird, wodurch zwar eine exakte Bilanzierung der Flüssigkeiten gewährleistet werden kann, die Effektivität der Blutbehandlung aber insgesamt negativ beeinflusst werden kann. Dies ist insbesondere deshalb kritisch, weil bei der Messung der Summe des Gewichts von Dialysat- und Substituatbeutel dieser Fehler nicht bemerkt werden kann.

Das erfindungsgemäße Verfahren und die erfindungsgemäße Vorrichtung sind für eine extrakorporale Blutbehandlungsvorrichtung bestimmt, mit der mehrere Behandlungsarten, beispielsweise eine Hämodialyse, Hämofiltration oder Hämodiafiltration durchgeführt werden können. Die extrakorporale Blutbehandlungsvorrichtung verfügt über eine Austauscheinheit, bei der es sich um einen Dialysator oder Filter handeln kann. Darüber hinaus weist die Blutbehandlungsvorrichtung drei Pumpen zum Fördern von Flüssigkeiten, d. h. Dialysat, Substituat und Filtrat, aus jeweils einem Behältnis bzw. in ein Behältnis auf. Dialysat und Substituat werden jeweils aus einem Behältnis und Filtrat in ein Behältnis gefördert. Bei dem Behältnis kann es sich um einen Beutel handeln, in dem Flüssigkeit bereitgestellt oder gesammelt wird. Des Weiteren weist die Blutbehandlungsvorrichtung Mittel zur Bestimmung der Summe des Gewichts von zwei Behältnissen und Mittel zur Bestimmung des Gewichts eines Behältnisses auf. Vorzugsweise sind die Mittel zur Bestimmung des Gewichts der Behältnisse Waagen. Die Mittel zur Bestimmung der Summe des Gewichts von zwei Behältnissen umfassen vorzugsweise nur eine Waage. Es können aber auch zwei Waagen verwendet werden, wobei die Summe des mit jeder Waage gemessenen Gewichts berechnet wird.

Eine alternative Ausführungsform sieht vor, dass die erste und dritte Pumpe, d. h. die Dialysat- und die Substituatpumpe, Flüssigkeit auch aus einem gemeinsamen Behältnis fördern können. Diese alternative Ausführungsform setzt aber voraus, dass Dialysat und Substituat die gleiche Flüssigkeit sind.

Das erfindungsgemäße Verfahren und die erfindungsgemäße Vorrichtung sehen für die Regelung der Pumpen neben der Bilanzierung der Flüssigkeiten in Abhängigkeit von den Gewichten der Behältnisse die Messung der Gewichtsab- bzw. -zunahme mindestens eines der Behältnisse in dem Zeitintervall vor, in dem die dem jeweiligen Behältnis zugeordnete Pumpe eine vorgegebene Anzahl von Umdrehungen oder Pumpenhüben ausführt. Wenn es sich um Rollenpumpen handelt, wird eine bestimmte Anzahl von Umdrehungen vorgegeben. Andernfalls wird eine bestimmte Anzahl von Pumpenhüben angenommen. Beispielsweise kann die Veränderung des Gewichts der Behältnisses für eine halbe oder eine Pumpenumdrehung bestimmt werden.

Aus der gemessenen Gewichtsab- bzw. -zunahme in dem bestimmten Zeitintervall wird die Förderrate der jeweiligen Pumpe ermittelt. Die Ermittlung der Gewichtsab- bzw. - zunahme pro Zeiteinheit erlaubt die genaue Bestimmung der tatsächlichen Förderrate einer oder mehrerer Pumpen. Vorzugsweise werden die tatsächlichen Förderraten der Dialysatpumpe, der Substituatpumpe und der Filtratpumpe überwacht.

Der Regelung liegt grundsätzlich die Soll-Förderrate der Pumpen, bei der es sich um die Dialysatrate, Filtratrate oder Substituatrate handeln kann, zugrunde, die als bekannt vorausgesetzt wird. Diese bei der Ansteuerung angenommene Förderrate, die sich bei einer vorgegebenen Anzahl von Umdrehungen pro Zeiteinheit der Pumpe einstellen sollte, wird mit der tatsächlichen Förderrate der Pumpe, d. h. der Ist-Förderrate, verglichen.

Die Differenz zwischen der Soll- und Ist-Förderrate wird bei der folgenden Ansteuerung der Pumpen berücksichtigt. Dies kann in der Praxis dadurch erfolgen, dass bei der Regelung der Pumpen ein Offset oder Korrekturfaktor berücksichtig wird, der sich aus dem Verhältnis von Soll-Förderrate zur Ist-Förderrate ergibt, im Folgenden Basiskorrekturfaktor genannt. Wenn dieser Basiskorrekturfaktor abhängig von der Soll-Förderrate ist, kann der Basiskorrekturfaktor für eine beliebige Anzahl verschiedener Soll-Förderraten bestimmt und bei der Ansteuerung der Pumpen berücksichtigt werden.

Mit dem erfindungsgemäßen Verfahren und der erfindungsgemäßen Vorrichtung werden also unabhängig von der eigentlichen Regelung schon bei Ansteuerung die systematischen Abweichungen der Flussraten berücksichtigt, die aus bauteilbedingten Abweichungen beispielsweise aufgrund der Toleranzen der geometrischen Abmessungen der Pumpen etc. und aus den Toleranzen der zugehörigen Schlauchleitungen resultieren. Es kann nun ein wesentlich kleinerer Regelbereich für die Pumpen festgelegt werden, weil die systematischen Abweichungen nicht mehr durch eine Regelung der Pumpen ausgeglichen werden müssen. Ein kleinerer Regelbereich bedeutet wiederum eine geringere Gefahr, dass ein zu großer Anteil des Fördervolumens einer Pumpe von einer anderen Pumpe gefördert wird und ermöglicht eine Verlängerung der Intervalle zwischen den Tests der Waagen. Insgesamt wird dadurch die Blutbehandlung effektiver. Ein kurzzeitiges Verlassen des eingeschränkten Toleranzbandes bei der Regelung der Pumpen kann toleriert werden, um z.B. Waagenstörungen besser ausgleichen zu können.

Eine bevorzugte Ausführungsform der Erfindung sieht die Bestimmung des Basiskorrekturfaktors sämtlicher Pumpen vor. Grundsätzlich ist es aber auch möglich, den Basiskorrekturfaktor nur für eine Pumpe oder zwei Pumpen zu bestimmen. Dann kann der systematische Fehler der beiden anderen Pumpen bzw. der anderen Pumpe aber nicht erfasst und für diese somit der Regelbereich nicht verkleinert werden. Die Einstellung eines besonders kleinen Regelbereichs für alle an der Bilanzierung beteiligten Pumpen ist also nur dann möglich, wenn der Basiskorrekturfaktor aller dieser Pumpen ermittelt wird.

Bei der bevorzugten Ausführungsform, bei der die Regelung von zwei Pumpen durch die Bestimmung der Gewichtsänderung auf nur einer Waage erfolgt, wird bei der Bestimmung des Basiskorrekturfaktors einer Pumpe die der selben Waage zugeordnete Pumpe angehalten. Dadurch kann die Veränderung des Gewichts des Behältnisses ausschließlich der fördernden Pumpe zugeordnet werden.

Bei einer Hämodiafiltration wird der zweiten Kammer der Austauscheinheit aus einem Dialysat-Beutel Dialysat zugeführt und Filtrat wird aus der zweiten Kammer der Austauscheinheit in einen Filtrat-Beutel abgeführt. Aus einem Substituat-Beutel wird Substituat dem extrakorporalen Blutkreislauf zugeführt. Dialysat wird mit der ersten Pumpe, Filtrat mit der zweiten Pumpe und Substituat mit der dritten Pumpe gefördert. Zur Bilanzierung der Flüssigkeiten werden die Pumpen derart geregelt, dass die Differenz zwischen der Gewichtsabnahme pro Zeiteinheit der Summe der Gewichte des Dialysats-Behältnisses und des Substituat-Behältnisses und der Gewichtszunahme pro Zeiteinheit des Filtrat-Behältnisses einem vorgegebenen Wert entspricht. Wenn der vorgegebene Wert 0 ist, wird dem Patienten weder Flüssigkeit zugeführt noch entzogen.

Eine bevorzugte Ausführungsform sieht vor, dass bei der Bestimmung des Basiskorrekturfaktors einer Pumpe der Betrag der Abweichung der Soll-Fördermenge dieser Pumpe von der Ist-Fördermenge bei der vorgegebenen Anzahl von Umdrehungen oder Pumpenhüben mit einem vorgegebenen Grenzwert für eine maximale Abweichung verglichen wird. Wenn der Betrag der Abweichung größer als der vorgegebene Grenzwert ist, wird auf einen Fehler geschlossen, wobei ein Fehlersignal erzeugt wird. Wenn ein Fehlersignal erzeugt wird, kann die Messung wiederholt werden. Wenn nicht der Betrag der Abweichung berechnet wird, kann zwischen einer positiven Abweichung nach oben und einer negativen Abweichung nach unten von einem Basiswert unterschieden werden. Beispielsweise kann für eine maximale Abweichung des Sollwerts vom Istwert in der Praxis +/- 0,3 g pro ½ Umdrehung der Pumpe vorgegeben werden. Für den Vergleich der Abweichung der Soll-Fördermenge von der Ist-Fördermenge kann auch ein oberer und unterer absoluter Grenzwert definiert werden.

Grundsätzlich ist es ausreichend, nur eine einzige Messung der Gewichtsab- bzw. - zunahme pro Zeiteinheit, d.h. pro Umdrehungen oder Pumpenhüben durchzuführen. Eine höhere Genauigkeit kann aber dadurch erzielt werden, dass mehrere Messungen durchgeführt werden und ein Mittelwert gebildet wird.

Von Vorteil ist, dass die für das erfindungsgemäße Verfahren und die erfindungsgemäße Vorrichtung erforderlichen Komponenten im Allgemeinen ohnehin in den bekannten Blutbehandlungsvorrichtungen vorhanden sind. Die Messung der Gewichtsab- bzw. - zunahme pro Zeiteinheit mit den für die Bilanzierung ohnehin vorhandenen Waagen erfordert daher keinen zusätzlichen gerätetechnischen Aufwand. Die Steuerung der einzelnen Komponenten kann von der zentralen Steuer- und Recheneinheit übernommen werden, die in jeder Blutbehandlungsvorrichtung vorhanden ist. Auch kann die zentrale Steuer- und Recheneinheit der Blutbehandlungsvorrichtung die erforderlichen Rechenoperationen ausführen. Die erfindungsgemäße Vorrichtung ist daher vorzugsweise Bestandteil der extrakorporalen Blutbehandlungsvorrichtung. Sie kann aber auch eine separate Baugruppe bilden.

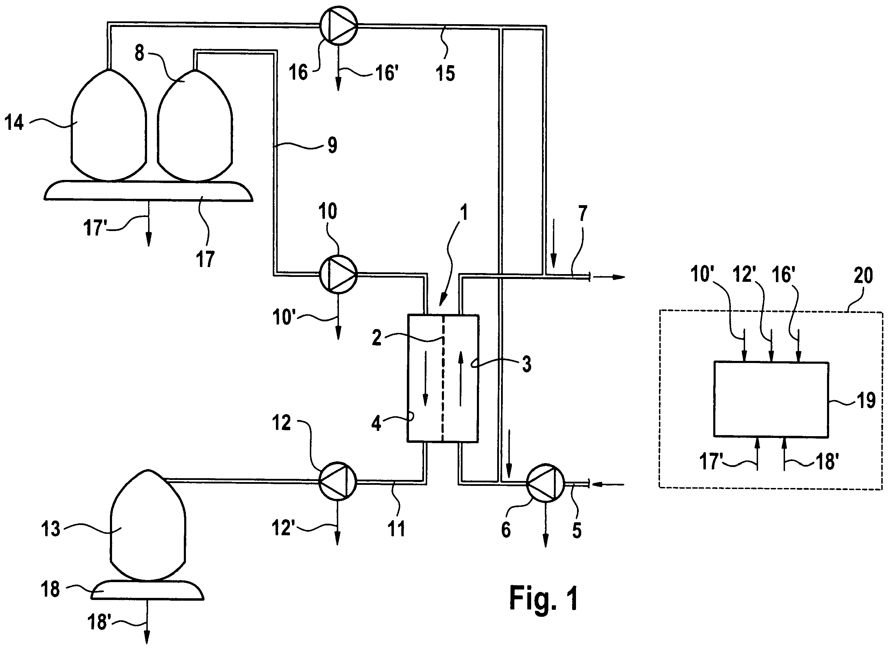

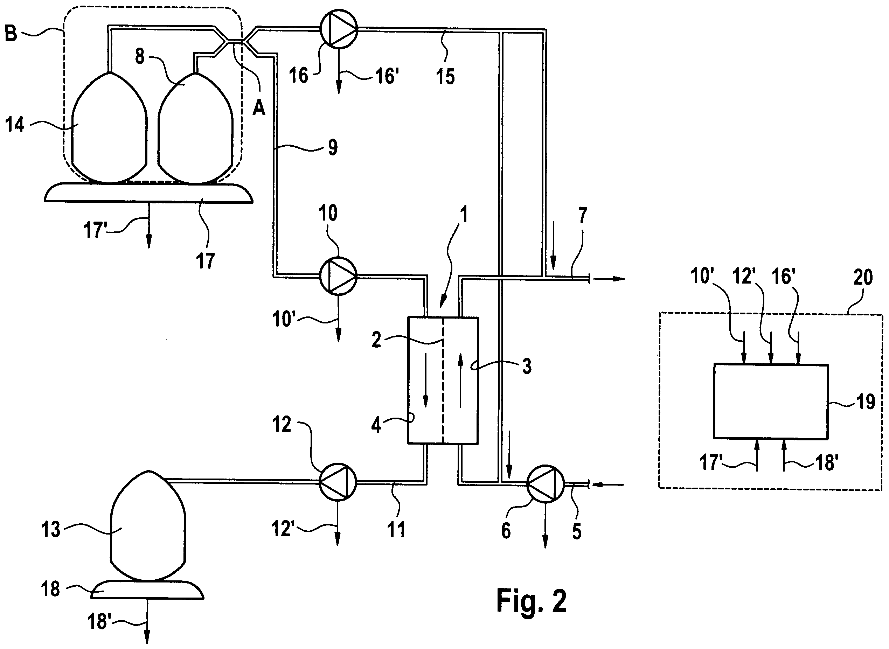

Im Folgenden wird ein Ausführungsbeispiel der Erfindung unter Bezugnahme auf die Zeichnungen im Einzelnen erläutert, die in stark vereinfachter schematischer Darstellung die wesentlichen Komponenten von zwei alternativen Ausführungsformen einer extrakorporalen Blutbehandlungsvorrichtung zeigen, bei denen die Bilanzierung der Flüssigkeiten mittels Waagen erfolgt. In den Figuren 1 und 2 sind die einander entsprechenden Teile mit den gleichen Bezugszeichen versehen.

Bei der extrakorporalen Blutbehandlungsvorrichtung handelt es sich insbesondere um eine für die akute Dialyse auf Intensivstationen bestimmte Dialysevorrichtung, mit der eine Hämodialyse, Hämofiltration oder Hämodiafiltration durchgeführt werden kann.

Die Blutbehandlungsvorrichtung verfügt über eine Austauscheinheit 1, die durch eine semipermeable Membran 2 in eine erste Kammer 3 und eine zweite Kammer 4 unterteilt ist. Die Austauscheinheit wird nachfolgend als Dialysator 1 bezeichnet. Die erste Kammer 3 des Dialysators 1 wird von Blut durchflossen. Das Blut wird über eine Blutzuführleitung 5 von dem Patienten mit einer Blutpumpe 6 in die erste Kammer 3 des Dialysators 1 gefördert und fließt über eine Blutabführleitung 7 aus der ersten Kammer 3 des Dialysators 1 zurück zum Patienten (Fig. 1).

Für die Blutbehandlung wird Dialysierflüssigkeit (Dialysat) in einem ersten Behältnis 8 bereitgestellt, bei dem es sich vorzugsweise um einen Beutel handelt. Aus dem Dialysat-Beutel 8 wird das Dialysat über eine Dialysatleitung 9, in die eine Dialysat-Pumpe 10 geschaltet ist, in die zweite Kammer 4 des Dialysators 1 gefördert. Aus der zweiten Kammer 4 des Dialysators 1 wird Filtrat durch eine Filtratleitung 11, in die eine Filtratpumpe 12 geschaltet ist, in ein Filtrat-Behältnis 13, insbesondere in einen Fitrat-Beutel, gefördert. Für die Dialysebehandlung wird Substituat in einem Substituat-Behältnis 14, insbesondere in einem Substituat-Beutel bereitgestellt. Von dem Substituat-Beutel 14 wird das Substituat über eine Substituat-Leitung 15, in die eine Substituat-Pumpe 16 geschaltet ist, dem extrakorporalen Blutkreislauf entweder stromauf der ersten Kammer 3 des Dialysators (Prädilution) oder stromab der ersten Kammer 3 des Dialysators 1 (Postdilution) zugeführt.

Bei der Dialysatleitung 9, der Filtratleitung 11 und der Substituatleitung 15, die mit dem Dialysat-Beutel 8 bzw. Filtrat-Beutel 13 bzw. Substituat-Beutel 14 verbunden sind, handelt es sich um Schlauchleitungen eines zur einmaligen Verwendung bestimmten Schlauchsystems (Disposable). Die Dialysat-Pumpe 10, die Filtrat-Pumpe 12 und die Substituat-Pumpe 16 sind peristaltische Pumpen, insbesondere Rollenpumpen, in die die Schlauchleitungen eingelegt sind. Die Rollenpumpen verfügen vorzugsweise über einen Schrittmotor, wobei die Anzahl der Schritte (Umdrehungen) die Fördermenge der Pumpe vorgibt. Jede Pumpe hat bei einer vollen bzw. halben Umdrehung eine bestimmte theoretische Fördermenge, auf die auch die eingelegte Schlauchleitung Einfluss hat. Die vom Hersteller angegebene Soll-Förderrate der Pumpe kann aber von der tatsächlichen Förderrate abweichen. Daher ist eine exakte Bilanzierung der Flüssigkeiten über die gesamte Behandlungszeit ohne Ausgleich dieser Abweichung nicht gewährleistet. Anstelle eines Schrittmotors kann auch ein anderer Motor eingesetzt werden. Beispielsweise kann ein Motor mit Bürsten oder ein bürstenloser Motor mit üblichem Taktgeber eingesetzt werden.

Zur exakten Bilanzierung der Flüssigkeiten ist eine Bilanziereinrichtung vorgesehen, die über eine erste Waage 17 und eine zweite Waage 18 verfügt. Die erste Waage 17 dient zum Wiegen des Dialysat-Beutels 8 und des Substituat-Beutels 14, während mit der zweiten Waage 18 der Filtrat-Beutel 13 gewogen wird. Die Waagen 17, 18 können unterschiedlich ausgebildet sein. Beispielsweise können die Beutel auf den Waagen liegen oder an den Waagen hängen. Die erste Waage 17 wiegt die Summe des Gewichts des Dialysat- und Substituat-Beutels 8, 14, während die zweite Waage nur das Gewicht des Filtrat-Beutels 13 wiegt. Ebenso können die Behälter 8 und 14 nicht gemeinsam auf der Waage 14, sondern auf zwei separaten Waagen platziert sein.

Zur Regelung der Dialysat-, Filtrat- und Substituat-Pumpe 10, 12, 16 verfügt die erfindungsgemäße Steuerungsvorrichtung über eine Regeleinheit 19, die Bestandteil der zentralen Steuer- und Recheneinheit 20 der extrakorporalen Blutbehandlungsvorrichtung sein kann. Die Steuer- und Recheneinheit 20 weist eine CPU zur Durchführung der Berechnungen und der Steuerung auf.

Die Regeleinheit 19 ist über Messleitungen 17', 18' mit der ersten Waage 17 und der zweiten Waage 18 verbunden. Über Steuerleitungen 10', 12' und 16' ist die Regeleinheit 19 mit der Dialysat-Pumpe 10, der Filtrat-Pumpe 12 und der Substituat-Pumpe 16 verbunden.

Zur Durchführung der Blutbehandlung wird zunächst über eine nicht dargestellte Eingabeeinheit eine bestimmte Förderrate für die Dialysat-Pumpe 10, die Filtrat-Pumpe 12 und die Substituat-Pumpe 16 vorgegeben. Zur Einstellung der Förderraten gibt die Regeleinheit 19 für die Schrittmotoren der Pumpen eine bestimmte Anzahl von Schritten (Umdrehungen) pro Zeiteinheit vor. Dabei wird angenommen, dass sich die gewünschte Förderrate auch exakt einstellt, was in der Praxis aber nicht der Fall ist.

Beispielsweise werden eine Dialysatrate von 2000 ml/h und eine Substituatrate von 900 ml/h und eine Netto-Ultrafiltrationsrate von 100 ml/h angenommen. Daraus ergibt sich eine Filtratrate von 3000 ml/h (50 ml/min). Da die tatsächlichen Förderraten der Pumpen von den Soll-Förderraten abweichen, wird in der Praxis die vorgegebene Netto-Ultrafiltrationsrate nicht erzielt. Es kann zu einer fehlerhaften Bilanzierung kommen.

Zur Bilanzierung der Flüssigkeiten regelt die Regeleinheit 19 die Förderraten der Dialysat-Pumpe 10, der Filtrat-Pumpe 12 und der Substituat-Pumpe 16. Dabei wird die Förderrate einer oder mehrerer Pumpen gezielt erhöht oder verringert, um den Fehler auszugleichen. Die Anpassung der Förderraten erfolgt innerhalb eines vorgegebenen Regelbereichs. Dabei soll die Förderrate einer oder mehrerer Pumpen nicht um einen Betrag erhöht oder verringert werden, der größer als ein bestimmter Grenzwert ist. Dieser Grenzwert kann ein prozentualer Anteil an der Soll-Förderrate sein, bspw. +/- 20 %.

Unter der Annahme, dass dem Patienten weder Flüssigkeit zugeführt noch entzogen werden soll, d.h. die Netto-Ultrafiltrationsrate ist 0, regelt die Regeleinheit 19 die Pumpen derart, dass die Summe der Gewichtsabnahme des Dialysat- und Substituat-Beutels 8, 14 in einer bestimmten Zeiteinheit, bspw. pro Minute oder Stunde, der Gewichtszunahme des Filtrat-Beutels 13 entspricht. Für den Fall, dass dem Patienten Flüssigkeit entzogen werden soll, regelt die Regeleinheit die Pumpen derart, dass der Betrag der Differenz der Gewichtsabnahme der Summe des Gewichts des Dialysat- und Substituat-Beutels 8, 14 und der Gewichtszunahme des Gewichts des Filtrat-Beutels 13 der Netto-Ultrafiltrationsrate entspricht.

Bei dem obigen Ausführungsbeispiel wird zur Erzielung der Netto-Ultrafiltrationsrate von 100 ml/h eine Filtratrate von 3000 ml/h (50 ml/min) eingestellt. Bei einem angenommenen Regelbereich für die Filtratpumpe von +/- 20 % kann die Filtratrate um maximal +/- 10 ml/min. bezogen auf den Sollwert erhöht bzw. verringert werden, um Fehler auszugleichen.

Zur Vermeidung einer fehlerhaften Bilanzierung ist es erforderlich, die Waagen 17, 18 in bestimmten Zeitintervallen zu testen. Die zeitlichen Intervalle zwischen den Tests richten sich nach dem Volumen, was unerkannt von der eigentlichen Bilanzierung dem Patienten zugeführt oder entzogen werden kann (Fehlbilanz). Wenn davon ausgegangen wird, dass eine Pumpe vor z.B. einer Leckage in der Mitte des Regelbereichs angesteuert, könnte die Fehlbilanzrate im angenommenen Fall +/- 20 % = +/- 10 ml/min der Soll-Förderrate entsprechen. Wenn die Fehlbilanz bspw. zwischen zwei Waagentests nicht größer als 500 ml sein soll, ist es bei dem vorliegenden Ausführungsbeispiel notwendig, alle 50 min einen Waagentest durchzuführen (500 ml / 10 ml/min = 50 min).

Die Funktionsweise der Regeleinheit 19 zur Steuerung der extrakoporalen Blutbehandlungsvorrichtung wird nachfolgend am Beispiel der Hämodiafiltration beschrieben. Die Dialysat- und Substituatpumpe 10, 16 fördern von einer gemeinsamen Waage 17. Die Erfindung sieht eine Verringerung des Regelbereichs vor. Bei dem vorliegenden Ausführungsbeispiel soll der Regelbereich bspw. nur +/- 8 % der Soll-Förderrate betragen.

Die zentrale Rechen- und Steuereinheit 20 der Blutbehandlungsvorrichtung erzeugt vorzugsweise vor der eigentlichen Blutbehandlung ein Steuersignal zur Einleitung der Bestimmung des Basiskorrekturfaktors für die an der Bilanzierung beteiligten Pumpen 10, 12, 16. Die Bestimmung des Basiskorrekturfaktors kann aber auch während der Behandlung oder Behandlungspause eingeleitet werden. Die Bestimmung des Basiskorrekturfaktors findet vorzugsweise während der Behandlung, z.B. 10 min nach Behandlungsstart statt, wenn die Dialysat-Pumpe 10, die Filtrat-Pumpe 12 und die Substituat-Pumpe 16 laufen.

Nach dem Empfang des Steuersignals stoppt die Regeleinheit 19 die Substituat-Pumpe 16. Die Förderrate der Filtrat-Pumpe 12 wird von der Regeleinheit dann so angepasst, dass sich wieder die gewünschte Netto-Ultrafiltrationsrate einstellt. Hierfür wird die Soll-Förderrate der Filtrat-Pumpe 12 um den Fluss der gestoppten Pumpe 16 reduziert.

Daraufhin wird die pro Umdrehung bzw. pro Hub von der Dialysat-Pumpe 10 geförderte Menge an Dialysat bestimmt, die nachfolgend als Schlagvolumen der Dialysat-Pumpe bezeichnet wird. Hierzu wird die Gewichtsabnahme des Dialysat-Beutels 8 in dem Zeitintervall erfasst, in dem die Dialysat-Pumpe eine bestimmte Anzahl von Umdrehungen bzw. Hüben ausgeführt hat. Die der Gewichtsabnahme in diesem Zeitintervall entsprechende Abnahme des Volumens an Dialysat dividiert durch die Anzahl der ausgeführten Umdrehungen bzw. Hübe entspricht dem zu messenden Schlagvolumen der Dialysat-Pumpe. Die Regeleinheit 19 führt vorzugsweise eine Vielzahl von aufeinander folgenden Messungen für eine halbe oder ganze Pumpenumdrehung durch, beispielsweise 20 Messungen, und berechnet den Mittelwert aus den ermittelten Schlagvolumina.

Anschließend setzt die Regeleinheit 19 wieder die Substituat-Pumpe 16 in Gang und hält nunmehr die Dialysat-Pumpe 10 an. Jetzt bestimmt die Regeleinheit 19 auf die gleiche Weise wie bei der Dialysat-Pumpe 10 das Schlagvolumen der Substituat-Pumpe 16. Nach Bestimmung des Schlagvolumens der Substituat-Pumpe 16 wird die Dialysat-Pumpe 16 wieder in Gang gesetzt. Auch bei der Ermittlung des Schlagvolumens der Substituat-Pumpe 16 wird die Soll-Förderrate der Filtrat-Pumpe 12 wieder von der Regeleinheit 19 um den Fluss der nun gestoppten Pumpe 10 reduziert, so dass sich die gewünschte Netto-Ultrafiltrationsrate einstellt.

Es ist auch möglich, dass die oben genannten angehaltenen Pumpen zur Bestimmung des Korrekturfaktors einer anderen Pumpe nicht gestoppt, sondern nur in ihrem Fluss auf ein Minimum reduziert werden. Dementsprechend kann dann der Fluss der Filtrat-Pumpe 12 um den reduzierten Fluss angepasst werden.

Die Ermittlung des Schlagvolumens der Filtrat-Pumpe 13 erfolgt während der Ermittlung des Schlagvolumens der Dialysat- und Substituat-Pumpe 10, 16. Hierzu wird in aufeinander folgenden Messungen jeweils die Gewichtszunahme des Filtrat-Beutels 13 innerhalb des Zeitintervalls erfasst, in dem die Filtrat-Pumpe 12 eine bestimmte Anzahl von Umdrehungen ausführt, wobei eine Mittelwertbildung der ermittelten Schlagvolumina erfolgt.

Nach der Bestimmung der Schlagvolumina der Pumpen 10, 12, 16 wird das bisher bei der Regelung angenommene Schlagvolumen, das dem vom Hersteller der Pumpen angegebenen Wert entspricht, mit dem ermittelten tatsächlichen Schlagvolumen der Pumpen ins Verhältnis gesetzt, um einen entsprechenden Regelfaktor, den Basiskorrekturfaktor zu bestimmen. Um den so bestimmten Basiskorrekturfaktor wird dann die "Nulllinie" für die Pumpensteuerung verschoben. Die Pumpenansteuerung erfolgt somit mit einem bestimmten "Offset". Dadurch wir der Anteil der systematischen Flussabweichungen, die sich aus den Toleranzen der Pumpen und des Einwegartikels (Disposables) ergeben, berücksichtigt, so dass ein kleinerer Regelbereich, beispielsweise +/- 8 % der Soll-Flussrate vorgegeben werden kann, um die zwischen den zyklischen Waagentests liegender Zeitintervalle verlängern zu können.

Der Basiskorrekturfaktor soll keinen größeren Offset als durch die bauteilbedingten Abweichungen maximal möglichen Fehler der Soll-Förderrate von mehr als bspw. +/- 15 % bewirken. Liegt der ermittelte Basiskorrekturfaktor beispielsweise zwischen +/- 15 % und +/- 20 %, dann wird er von der Regeleinheit 19 auf +/- 15 % begrenzt. Liegt der ermittelte Offset bei mehr als +/- 20 %, gilt die Ermittlung des Schlagvolumens als nicht erfolgreich und kann vom Anwender wiederholt werden. Die Regeleinheit 19 erzeugt in diesem Fall ein Fehlersignal.

Während der Bestimmung des Basiskorrekturfaktors der Pumpen als gestört identifizierte Schlagvolumina werden nicht zur Bestimmung des Basiskorrekturfaktors herangezogen. Sollte ein Fehler vorliegen, kann die Messung der Gewichtsab- bzw. -zunahme um die Anzahl der gestörten Schlagvolumina verlängert werden. Wenn auch auf diese Weise mehr als eine bestimmte Anzahl von Pumpenumdrehungen für die Bestimmung des Basiskorrekturfaktors erforderlich ist, bspw. 25 Pumpenumdrehungen, gilt die Kalibrierung als nicht bestanden und kann vom Anwender wiederholt werden. Auch in diesem Fall erzeugt die Regeleinheit 19 ein Fehlersignal. Um eventuell auftretende Langzeitabweichungen der Schlagvolumina auszugleichen, kann der Basiskorrekturfaktor über einen separaten Algorithmus auch nach Abschluss der Kalibrierung noch nachgeführt werden.

Die Bestimmung der Schlagvolumina muss während einer laufenden Behandlung nicht wiederholt werden. Versuche im Labor haben ergeben, dass das Schlagvolumen der verwendeten Pumpen nach einer Einlaufdauer von etwa 5 Minuten über die folgenden 70 Stunden nur noch kaum messbar driftet. Während der Bestimmung der Schlagvolumina können die Flussraten der Pumpen auf einen oberen Wert, der beispielsweise bei maximal 2.000 ml/h liegen kann, begrenzt werden, um die Genauigkeit der Bestimmung des Basiskorrekturfaktors zu erhöhen.

Anhand des folgenden Beispiels soll nochmals verdeutlicht werden, dass der Abstand zwischen den zyklischen Waagentests dann deutlich erhöht werden kann, wenn nach der Bestimmung der Schlagvolumina der Pumpen der erlaubte Regelbereich für die Filtrat-Pumpe verringert wird.

### Beispiel:

Verfahren: CVVHDF
Dialysatrate: 2.000 ml/h
Substituatrate: 900 ml/h
Netto-Ultrafiltrationsrate: 100 ml/h

Zur Einstellung der gewünschten Netto-Ultrafiltrationsrate muss von der Regeleinheit eine Filtratrate von 3.000 ml/h = 50 ml/min vorgegeben werden. Es wird ein Filtratregelbereich von +/- 8 % = +/- 4 ml/min vorgegeben. Da die Pumpe nun im Normalfall genau in der Mitte des Regelbereiches angesteuert wird, kann die z.B. durch eine Leckage resultierende Fehlbilanzrate nur den einseitigen Regelbereich von 8 % = 4 ml/min betragen. Wenn als maximale Fehlbilanz 500 ml erlaubt sind, ergibt sich ein zyklischer Waagentest, der alle 125 min (500 ml / 4 ml/min = 125) auszuführen ist. Es zeigt sich, dass das zwischen den Waagentests liegende Intervall größer ist als bei einem Regelbereich von +/- 20 %, bei dem das Testintervall 50 min ist.

Wenn das erfindungsgemäße Verfahren und die erfindungsgemäße Vorrichtung bei einer Hämodialyse Verwendung finden, müssen nur die Schlagvolumina der Dialysat- und Filtrat-Pumpe bestimmt werden, da für die Hämodialyse die Substituat-Pumpe nicht betrieben wird.

Wenn das erfindungsgemäße Verfahren und die erfindungsgemäße Vorrichtung bei einer Hämofiltration (CVVH) Verwendung finden, müssen nur die Schlagvolumina der Substituat- und Filtrat-Pumpe bestimmt werden, weil für die Hämofiltration die Dialysat-Pumpe nicht betrieben wird.

Fig. 2 zeigt eine alternative Ausführungsform der extrakorporalen Blutbehandlungsvorrichtung, die sich von dem ersten Ausführungsbeispiel nur dadurch unterscheidet, dass die beiden Leitungen 9 und 15 von den Beuteln 8 und 14 über einen gemeinsamen Leitungsabschnitt A verfügen. Beispielsweise können die beiden Leitungen 9, 15 mit einem nicht dargestellten ersten Y-Verbindungsstück zu dem gemeinsamen Leitungsabschnitt A, der beispielweise eine Länge von 50 cm haben kann, zusammengeführt und mit einem ebenfalls nicht dargestellten zweiten Y-Verbindungsstück wieder aufgeteilt werden. Die Zusammensetzung des Dialysats und Substituats in den Beuteln 8 bzw. 14 ist in diesem Fall identisch. Die beiden Beutel 8, 14 wirken dabei wie ein einziger Beutel. Daher kann anstelle der beiden Beutel 8, 14 Dialysat und Substituat auch in einem einzigen Beutel bereitgestellt werden, an den die Leitungen 9 und 15 angeschlossen sind. Dieses einzige Behältnis B, das die Behältnisse 8 und 14 ersetzt, ist in Fig. 2 als weitere alternative Ausführungsform in gestrichelten Linien gezeigt.

Die alternative Ausführungsform mit den beiden Beuteln und dem gemeinsamen Leitungsabschnitt hat den Handhabungsvorteil, dass beide Beutel 8 und 14 unabhängig von den unterschiedlichen Flussraten der Pumpen 10 und 16 gleichzeitig leer werden. Bei der Bereitstellung der Flüssigkeit in nur einem Beutel, stellt sich das Problem der gleichzeitigen Entleerung überhaupt nicht.

## Patentansprüche

1. Verfahren zum Steuern einer extrakorporalen Blutbehandlungsvorrichtung, die aufweist:
eine Austauscheinheit (1), die durch eine semipermeable Membran (2) in eine erste Kammer (3) und eine zweite Kammer (4) unterteilt ist, wobei die erste Kammer Teil eines extrakorporalen Blutkreislaufs und die zweite Kammer Teil eines Flüssigkeitssystems ist,
eine erste Pumpe (10) zum Fördern einer Flüssigkeit aus einem ersten Behältnis (8) mit einer ersten Förderrate in die zweite Kammer der Austauscheinheit,
eine zweite Pumpe (12) zum Fördern einer Flüssigkeit aus der zweiten Kammer der Austauscheinheit mit einer zweiten Förderrate in ein zweites Behältnis (13),
eine dritte Pumpe (16) zum Fördern einer Flüssigkeit mit einer dritten Förderrate aus einem dritten Behältnis (14) oder dem ersten Behältnis in den extrakorporalen Blutkreislauf,
erste Mittel (16) zur Bestimmung der Summe der Gewichte des ersten und dritten Behältnisses bzw. des Gewichts des ersten Behältnisses,
zweite Mittel (18) zur Bestimmung des Gewichts des zweiten Behältnisses,
wobei die Pumpen (10, 12, 16) zur Bilanzierung der Flüssigkeiten in Abhängigkeit von der Summe der Gewichte des ersten und dritten Behältnisses bzw. des Gewichts des ersten Behältnisses einerseits und des Gewichts des zweiten Behältnisses andererseits geregelt werden,
**dadurch gekennzeichnet, dass**
die Gewichtsabnahme oder Gewichtszunahme mindestens eines der Behältnisse (8, 13, 14) in dem Zeitintervall gemessen wird, in dem die dem jeweiligen Behältnis zugeordnete Pumpe (10, 12, 16) eine vorgegebene Anzahl von Umdrehungen oder Pumpenhübe ausführt,
aus der gemessenen Gewichtsabnahme bzw. Gewichtszunahme in dem bestimmten Zeitintervall die Fördermenge der jeweiligen Pumpe (10, 12, 16) bei der vorgegebenen Anzahl von Umdrehungen oder Pumpenhüben ermittelt wird, und
die bei der Ansteuerung der jeweiligen Pumpe (10, 12, 16) angenommene Soll-Fördermenge der Pumpe bei der vorgegebenen Anzahl von Umdrehungen oder Pumpenhüben mit der gemessenen Fördermenge der Pumpe bei der vorgegebenen Anzahl von Umdrehungen oder Pumpenhüben verglichen wird, und
der Ansteuerung der Pumpen (10, 12, 16) die Abweichung der angenommenen Soll-Fördermenge der jeweiligen Pumpe bei der vorgegebenen Anzahl von Umdrehungen oder Pumpenhüben von der gemessenen Fördermenge der Pumpe bei der vorgegebenen Anzahl von Umdrehungen oder Pumpenhüben zugrunde gelegt wird.

2. Verfahren nach Anspruch 1, **dadurch gekennzeichnet, dass** die Pumpen zur Bilanzierung der Flüssigkeiten derart geregelt werden, dass die Differenz zwischen der Gewichtsabnahme pro Zeiteinheit der Summe der Gewichte des ersten und dritten Behältnisses (8, 14) und der Gewichtszunahme pro Zeiteinheit des zweiten Behältnisses (13) einem vorgegebenen Wert entspricht.

3. Verfahren nach Anspruch 1 oder 2, **dadurch gekennzeichnet, dass** die Gewichtsabnahme des ersten Behältnisses (8) in dem Zeitintervall gemessen wird, in dem die erste Pumpe (10) eine vorgegebene Anzahl von Umdrehungen oder Pumpenhübe ausführt, und aus der gemessenen Gewichtsabnahme die Fördermenge der ersten Pumpe bei der vorgegebenen Anzahl von Umdrehungen oder Pumpenhüben ermittelt wird,
die Gewichtszunahme des zweiten Behältnisses (13) in dem Zeitintervall gemessen wird, in dem die zweite Pumpe (12) eine vorgegebene Anzahl von Umdrehungen oder Pumpenhübe ausführt, und aus der gemessenen Gewichtszunahme die Fördermenge der zweiten Pumpe bei der vorgegebenen Anzahl von Umdrehungen oder Pumpenhüben ermittelt wird,
die Gewichtsabnahme des dritten Behältnisses (14) in dem Zeitintervall gemessen wird, in dem die dritte Pumpe (16) eine vorgegebene Anzahl von Umdrehungen oder Pumpenhübe ausgeführt hat, und aus der gemessenen Gewichtsabnahme die Fördermenge der dritten Pumpe bei der vorgegebenen Anzahl von Umdrehungen oder Pumpenhüben ermittelt wird, und
dass die bei der Ansteuerung der Pumpen (10, 12, 16) angenommenen Fördermengen der Pumpen bei der vorgegebenen Anzahl von Umdrehungen oder Pumpenhüben mit den gemessenen Fördermengen der Pumpen bei der vorgegebenen Anzahl von Umdrehungen oder Pumpenhüben verglichen werden, und der Ansteuerung der Pumpen die Abweichung der angenommenen Fördermengen der Pumpen bei der vorgegebenen Anzahl von Umdrehungen oder Pumpenhüben von den gemessenen Fördermengen der Pumpen bei der vorgegebenen Anzahl von Umdrehungen oder Pumpenhüben zugrunde gelegt wird.

4. Verfahren nach einem der Ansprüche 1 bis 3, **dadurch gekennzeichnet, dass** die ersten Mittel (17) zur Bestimmung der Summe der Gewichte des ersten und dritten Behältnisses eine erste Waage zum gleichzeitigen Wiegen des ersten und dritten Behältnisses (8, 14) umfassen und die zweiten Mittel zur Bestimmung des Gewichts des zweiten Behältnisses eine zweite Waage zum Wiegen des zweiten Behältnisses umfassen.

5. Verfahren nach Anspruch 4, **dadurch gekennzeichnet, dass** für die Ermittlung der Gewichtsabnahme des ersten Behältnisses (8) die dritte Pumpe (16) in dem Zeitintervall, in dem die erste Pumpe die vorgegebene Anzahl von Umdrehungen oder Pumpenhübe ausführt, angehalten oder die Flussrate der dritten Pumpe reduziert wird.

6. Verfahren nach Anspruch 4 der 5, **dadurch gekennzeichnet, dass** für die Ermittlung der Gewichtsabnahme des dritten Behältnisses (14) die erste Pumpe (10) in dem Zeitintervall, in dem die dritte Pumpe eine vorgegebene Anzahl von Umdrehungen oder Pumpenhübe ausführt, angehalten oder die Flussrate der ersten Pumpe reduziert wird.

7. Verfahren nach einem der Ansprüche 1 bis 6, **dadurch gekennzeichnet, dass** die vorgegebene Soll-Förderrate mindestens einer der Pumpen (10, 12, 16) innerhalb eines vorgegebenen Regelbereichs um einen bestimmten Betrag, der kleiner als ein vorgegebener Grenzwert ist, erhöht oder verringert wird, so dass die Differenz zwischen der Gewichtsabnahme pro Zeiteinheit der Summe der Gewichte des ersten und dritten Behältnisses (8, 14) und der Gewichtszunahme pro Zeiteinheit des zweiten Behältnisses (13) einem vorgegebenen Wert entspricht

8. Verfahren nach Anspruch 7, **dadurch gekennzeichnet, dass** der vorgegebene Grenzwert ein vorgegebener prozentualer Betrag der vorgegebenen Soll-Förderrate der jeweiligen Pumpe (10, 12, 16) ist.

9. Verfahren nach einem der Ansprüche 1 bis 8, **dadurch gekennzeichnet, dass** die Abweichung der angenommenen Fördermenge der jeweiligen Pumpe (10, 12, 16) bei der vorgegebenen Anzahl von Umdrehungen oder Pumpenhüben von der gemessenen Fördermenge der Pumpe bei der vorgegebenen Anzahl von Umdrehungen oder Pumpenhüben mit einem vorgegebenen Grenzwert verglichen wird, wobei ein Fehlersignal erzeugt wird, wenn die Abweichung größer als der Grenzwert ist.

10. Blutbehandlungsvorrichtung mit
einer ersten Pumpe (10) zum Fördern einer Flüssigkeit aus einem ersten Behältnis (8) mit einer ersten Förderrate in die zweite Kammer (4) einer Austauscheinheit (1), die durch eine semipermeable Membran (2) in eine erste Kammer (3) und die zweite Kammer (4) unterteilt ist, wobei die erste Kammer (2) Teil eines extrakorporalen Blutkreislaufs und die zweite Kammer (4) Teil eines Flüssigkeitssystems ist,
einer zweiten Pumpe (12) zum Fördern einer Flüssigkeit aus der zweiten Kammer (4) der Austauscheinheit (1) mit einer zweiten Förderrate in ein zweites Behältnis (13),
einer dritten Pumpe (16) zum Fördern einer dritten Flüssigkeit mit einer dritten Förderrate aus einem dritten Behältnis (14) oder aus dem ersten Behältnis (8) in den extrakorporalen Blutkreislauf,
ersten Mitteln (17) zur Bestimmung der Summe der Gewichte des ersten und dritten Behältnisses (8, 14) bzw. des Gewichts des ersten Behältnisses (8),
zweiten Mitteln (18) zur Bestimmung des Gewichts des zweiten Behältnisses (13), und
einer Regeleinheit (19) zum Regeln der Förderraten der Pumpen, die derart ausgebildet ist, dass die Flüssigkeiten in Abhängigkeit von der Summe der Gewichte des ersten und dritten Behältnisses (8, 14) bzw. des Gewichts des ersten Behältnisses (8) einerseits und des Gewichts des zweiten Behältnisses (13) andererseits bilanziert werden,
**dadurch gekennzeichnet, dass** die Regeleinheit (19) derart ausgebildet ist, dass
die Gewichtsabnahme oder Gewichtszunahme mindestens eines der Behältnisse (8, 13, 14) in dem Zeitintervall gemessen wird, in dem die dem jeweiligen Behältnis zugeordnete Pumpe (10, 12, 16) eine vorgegebene Anzahl von Umdrehungen oder Pumpenhübe ausführt,
aus der gemessenen Gewichtsabnahme bzw. Gewichtszunahme in dem Zeitintervall die Fördermenge der jeweiligen Pumpe (10, 12, 16) bei der vorgegebenen Anzahl von Umdrehungen oder Pumpenhüben ermittelt wird, und die bei der Ansteuerung der jeweiligen Pumpe angenommene Soll-Fördermenge der Pumpe bei der vorgegebenen Anzahl von Umdrehungen oder Pumpenhüben mit der gemessenen Fördermenge der Pumpe bei der vorgegebenen Anzahl von Umdrehungen oder Pumpenhüben verglichen wird, und
der Ansteuerung der Pumpen (10, 12, 16) die Abweichung der angenommenen Soll-Fördermenge der jeweiligen Pumpe bei der vorgegebenen Anzahl von Umdrehungen oder Pumpenhüben von der gemessenen Fördermenge der Pumpe bei der vorgegebenen Anzahl von Umdrehungen oder Pumpenhüben zugrunde gelegt wird.

11. Vorrichtung nach Anspruch 10, **dadurch gekennzeichnet, dass** die Regeleinheit (19) derart ausgebildet ist, dass zur Bilanzierung der Flüssigkeiten die Förderraten der Pumpen (10, 12, 16) derart eingestellt werden, dass die Differenz zwischen der Gewichtsabnahme pro Zeiteinheit der Summe der Gewichte des ersten und dritten Behältnisses (8, 14) und der Gewichtszunahme pro Zeiteinheit des zweiten Behältnisses (13) einem vorgegebenen Wert entspricht.

12. Vorrichtung nach Anspruch 10 oder 11, **dadurch gekennzeichnet, dass** die Regeleinheit (19) derart ausgebildet ist, dass
die Gewichtsabnahme des ersten Behältnisses (8) in dem Zeitintervall gemessen wird, in dem die erste Pumpe (10) eine vorgegebene Anzahl von Umdrehungen oder Pumpenhübe ausführt, und aus der gemessenen Gewichtsabnahme die Fördermenge der ersten Pumpe (10) bei der vorgegebenen Anzahl von Umdrehungen oder Pumpenhüben ermittelt wird,
die Gewichtszunahme des zweiten Behältnisses (13) in dem Zeitintervall gemessen wird, in dem die zweite Pumpe (12) eine vorgegebene Anzahl von Umdrehungen oder Pumpenhübe ausführt, und aus der gemessenen Gewichtszunahme die Fördermenge der zweiten Pumpe (12) bei der vorgegebenen Anzahl von Umdrehungen oder Pumpenhüben ermittelt wird,
die Gewichtsabnahme des dritten Behältnisses (14) in dem Zeitintervall gemessen wird, in dem die dritte Pumpe (16) eine vorgegebene Anzahl von Umdrehungen oder Pumpenhübe ausgeführt hat, und aus der gemessenen Gewichtsabnahme die Fördermenge der dritten Pumpe (16) bei der vorgegebenen Anzahl von Umdrehungen oder Pumpenhüben ermittelt wird, und
dass die bei der Ansteuerung der Pumpen (10, 12, 16) angenommenen Fördermengen der Pumpen bei der vorgegebenen Anzahl von Umdrehungen oder Pumpenhüben mit den gemessenen Fördermengen der Pumpen bei der vorgegebenen Anzahl von Umdrehungen oder Pumpenhüben verglichen werden, und der Ansteuerung der Pumpen die Abweichung der angenommenen Fördermengen der Pumpen bei der vorgegebenen Anzahl von Umdrehungen oder Pumpenhüben von den gemessenen Fördermengen der Pumpen bei der vorgegebenen Anzahl von Umdrehungen oder Pumpenhüben zugrunde gelegt wird.

13. Vorrichtung nach einem der Ansprüche 10 bis 12, **dadurch gekennzeichnet, dass** die ersten Mittel (17) zur Bestimmung der Summe der Gewichte des ersten und dritten Behältnisses (8, 14) eine erste Waage (17) zum gleichzeitigen Wiegen des ersten und dritten Behältnisses umfassen und die zweiten Mittel zur Bestimmung des Gewichts des zweiten Behältnisses (13) eine zweite Waage (18) zum Wiegen des zweiten Behältnisses umfassen.

14. Vorrichtung nach Anspruch 13, **dadurch gekennzeichnet, dass** die Regeleinheit (19) derart ausgebildet ist, dass für die Ermittlung der Gewichtsabnahme des ersten Behältnisses (8) die dritte Pumpe (16) in dem Zeitintervall, in dem die erste Pumpe (10) die vorgegebene Anzahl von Umdrehungen oder Pumpenhübe ausführt, angehalten oder die Flussrate der dritten Pumpe reduziert wird.

15. Vorrichtung nach Anspruch 13 oder 14, **dadurch gekennzeichnet, dass** die Regeleinheit (19) derart ausgebildet ist, dass für die Ermittlung der Gewichtsabnahme des dritten Behältnisses (14) die erste Pumpe (10) in dem Zeitintervall, in dem die dritte Pumpe (16) eine vorgegebene Anzahl von Umdrehungen oder Pumpenhübe ausführt, angehalten oder die Flussrate der ersten Pumpe reduziert wird.

16. Vorrichtung nach einem der Ansprüche 10 bis 15, **dadurch gekennzeichnet, dass** die Regeleinheit (19) derart ausgebildet ist, dass die vorgegebene Soll-Förderrate mindestens einer der Pumpen (10, 12, 16) innerhalb eines vorgegebenen Regelbereichs um einen bestimmten Betrag, der kleiner als ein vorgegebener Grenzwert ist, erhöht oder verringert wird, so dass die Differenz zwischen der Gewichtsabnahme pro Zeiteinheit der Summe der Gewichte des ersten und dritten Behältnisses (8, 14) und der Gewichtszunahme pro Zeiteinheit des zweiten Behältnisses (13) einem vorgegebenen Wert entspricht

17. Vorrichtung nach Anspruch 16, **dadurch gekennzeichnet, dass** die Regeleinheit (19) derart ausgebildet ist, dass der vorgegebene Grenzwert ein vorgegebener prozentualer Betrag der vorgegebenen Soll-Förderrate der jeweiligen Pumpe ist.

18. Vorrichtung nach einem der Ansprüche 10 bis 17, **dadurch gekennzeichnet, dass** die Regeleinheit (19) derart ausgebildet ist, dass die Abweichung der angenommenen Fördermenge der jeweiligen Pumpe (10, 12, 16) bei der vorgegebenen Anzahl von Umdrehungen oder Pumpenhüben von der gemessenen Fördermenge der Pumpe bei der vorgegebenen Anzahl von Umdrehungen oder Pumpenhüben mit einem vorgegebenen Grenzwert verglichen wird, wobei ein Fehlersignal erzeugt wird, wenn die Abweichung größer als der Grenzwert ist.

## Claims

1. A method for controlling an extracorporeal blood treatment apparatus which comprises:
an interchangeable unit (1), which is divided by a semi-permeable membrane (2) into a first chamber (3) and a second chamber (4), wherein the first chamber is part of an extracorporeal blood circuit and the second chamber is part of a fluid system,
a first pump (10) for conveying a fluid from a first container (8) at a first delivery rate into the second chamber of the interchangeable unit,
a second pump (12) for conveying a fluid from the second chamber of the interchangeable unit at a second delivery rate into a second container (13),
a third pump (16) for conveying a fluid at a third delivery rate from a third container (14) or the first container into the extracorporeal blood circuit,
first means (17) for determining the sum of the weights of the first and third container or the weight of the first container,
second means (18) for determining the weight of the second container,
wherein the pumps (10, 12, 16) for balancing the fluids are regulated as a function of the sum of the weights of the first and third container or the weight of the first container on the one hand and the weight of the second container on the other hand,
**characterised in that**
the weight reduction or weight increase of at least one of the containers 8, 13, 14) is measured in the time interval in which the pump (10, 12, 16) assigned to the respective container performs a preset number of revolutions or pumps strokes,
the delivery quantity of the respective pump (10, 12, 16) at the preset number of revolutions or pump strokes is ascertained from the measured weight reduction or weight increase in the specific time interval, and
the setpoint delivery quantity of the pump at the preset number of revolutions or pump strokes adopted in the drive of the respective pump (10, 12, 16) is compared with the measured delivery quantity of the pump at the preset number of revolutions or pump strokes, and
the drive of the pumps (10, 12, 16) is based on the deviation of the adopted setpoint delivery quantity of the respective pump at the preset number of revolutions or pump strokes from the measured delivery quantity of the pump at the preset number of revolutions or pump strokes.

2. The method according to claim 1, **characterised in that** the pumps for balancing the fluids are controlled in such a way that the difference between the weight reduction per unit of time of the sum of the weights of the first and third container (8, 14) and the weight increase per unit of time of the second container (13) corresponds to a preset value.

3. The method according to claim 1 or 2, **characterised in that** the weight reduction of the first container (8) is measured in the time interval in which the first pump (10) performs a preset number of revolutions or pump strokes, and the delivery quantity of the first pump at the preset number of revolutions or pump strokes is ascertained from the measured weight reduction,
the weight increase of the second container (13) is measured in the time interval in which the second pump (12) performs a preset number of revolutions or pump strokes, and the delivery quantity of the second pump at the preset number of revolutions or pump strokes is ascertained from the measured weight increase,
the weight reduction of the third container (14) is measured in the time interval in which the third pump (16) performs a preset number of revolutions or pump strokes, and the delivery quantity of the third pump at the preset number of revolutions or pump strokes is ascertained from the measured weight reduction, and
the delivery quantities of the pumps (10, 12, 16) at the preset number of revolutions or pump strokes adopted in the drive of the pumps are compared with the measured delivery quantities of the pumps at the preset number of revolutions or pump strokes, and the drive of the pumps is based on the deviation of the adopted delivery quantities of the pumps at the preset number of revolutions or pump strokes from the measured delivery quantities of the pumps at the preset number of revolutions or pump strokes.

4. The method according to any one of claims 1 to 3, **characterised in that** the first means (17) for determining the sum of the weights of the first and third container comprise a first balance for the simultaneous weighing of the first and third container (8, 14) and the second means for determining the weight of the second container comprise a second balance for weighing the second container.

5. The method according to claim 4, **characterised in that**, for the determination of the weight reduction of the first container (8), the third pump (16) is stopped or the flow rate of the third pump is reduced in the time interval in which the first pump performs the preset number of revolutions or pump strokes.

6. The method according to claim 4 or 5, **characterised in that**, for the determination of the weight reduction of the third container (14), the first pump (10) is stopped or the flow rate of the first pump is reduced in the time interval in which the third pump performs a preset number of revolutions or pump strokes.

7. The method according to any one of claims 1 to 6, **characterised in that** the preset setpoint delivery rate of at least one of the pumps (10, 12, 16) is increased or reduced within a preset control range by a specific amount which is less than a preset limiting value, so that the difference between the weight reduction per unit of time of the sum of the weights of the first and third container (8, 14) and the weight increase per unit of time of the second container (13) corresponds to a preset value.

8. The method according to claim 7, **characterised in that** the preset limiting value is a preset percentage of the preset setpoint delivery rate of the respective pump (10, 12, 16).

9. The method according to any one of claims 1 to 8, **characterised in that** the deviation of the adopted delivery quantity of the respective pump (10, 12, 16) at the preset number of revolutions or pump strokes from the measured delivery quantity of the pump at the preset number of revolutions or pump strokes is compared with a preset limiting value, an error signal being generated when the deviation is greater than the limiting value.

10. Blood treatment apparatus comprising
a first pump (10) for conveying a fluid from a first container (8) at a first delivery rate into the second chamber (4) of an interchangeable unit (1) which is divided by a semi-permeable membrane (2) into a first chamber (3) and a second chamber (4), wherein the first chamber (2) is part of an extracorporeal blood circuit and the second chamber (4) is part of a fluid system,
a second pump (12) for conveying a fluid from the second chamber (4) of the interchangeable unit (1) at a second delivery rate into a second container (13),
a third pump (16) for conveying a third fluid at a third delivery rate from a third container (14) or from the first container (8) into the extracorporeal blood circuit,
first means (17) for determining the sum of the weights of the first and third container (8, 14) or the weight of the first container (8),
second means (18) for determining the weight of the second container (13),
a control unit (19) for controlling the delivery rates of the pumps, which is designed in such a way that the fluids are balanced as a function of the sum of the weights of the first and third container (8, 14) or the weight of the first container (8) on the one hand and the weight of the second container (13) on the other hand,
**characterised in that** the control unit (19) is designed in such a way that
the weight reduction or weight increase of at least one of the containers (8, 13,14) is measured in the time interval in which the pump (10, 12, 16) assigned to the respective container performs a preset number of revolutions or pump strokes,
the delivery quantity of the respective pump (10, 12, 16) at the preset number of revolutions or pump strokes is ascertained from the measured weight reduction or weight increase in the time interval, and
the setpoint delivery quantity of the pumps at the preset number of revolutions or pump strokes adopted in the drive of the respective pump is compared with the measured delivery quantity of the pump at the preset number of revolutions or pump strokes, and
the drive of the pumps (10, 12, 16) is based on the deviation of the adopted setpoint delivery quantity of the respective pump at the preset number of revolutions or pump strokes from the measured delivery quantity of the pump at the preset number of revolutions or pump strokes.

11. The device according to claim 10, **characterised in that** the control unit (19) is designed in such a way that, in order to balance the fluids, the delivery rates of the pumps (10, 12, 16) are set in such a way that the difference between the weight reduction per unit of time of the sum of the weights of the first and third container (8, 14) and the weight increase per unit of time of the second container (13) corresponds to a preset value.

12. The device according to claim 10 or 11, **characterised in that** the control unit (19) is designed in such a way that
the weight reduction of the first container (8) is measured in the time interval in which the first pump (10) performs a preset number of revolutions or pump strokes, and the delivery quantity of the first pump (10) at the preset number of revolutions or pump strokes is ascertained from the measured weight reduction,
the weight increase of the second container (13) is measured in the time interval in which the second pump (12) performs a preset number of revolutions or pump strokes, and the delivery quantity of the second pump (12) at the preset number of revolutions or pump strokes is ascertained from the measured weight increase,
the weight reduction of the third container (14) is measured in the time interval in which the third pump (16) performs a preset number of revolutions or pump strokes, and the delivery quantity of the third pump (16) at the preset number of revolutions or pump strokes is ascertained from the measured weight reduction, and
the delivery quantities of the pumps at the preset number of revolutions or pump strokes adopted in the drive of the pumps (10, 12, 16) are compared with the measured delivery quantities of the pumps at the preset number of revolutions or pump strokes, and the drive of the pumps is based on the deviation of the adopted delivery quantities of the pumps at the preset number of revolutions or pump strokes from the measured delivery quantities of the pumps at the preset number of revolutions or pump strokes.

13. The device according to any one of claims 10 to 12, **characterised in that** the first means (17) for determining the sum of the weights of the first and third container (8, 14) comprise a first balance (17) for the simultaneous weighing of the first and third container and the second means for determining the weight of the second container (13) comprise a second balance (18) for weighing the second container.

14. The device according to claim 13, **characterised in that** the control unit (19) is designed in such a way that, for the determination of the weight reduction of the first container (8), the third pump (16) is stopped or the flow rate of the third pump is reduced in the time interval in which the first pump (10) performs a preset number of revolutions or pump strokes.

15. The device according to claim 13 or 14, **characterised in that** the control unit (19) is designed in such a way that, for the determination of the weight reduction of the third container (14), the first pump (10) is stopped or the flow rate of the first pump is reduced in the time interval in which the third pump (16) performs a preset number of revolutions or pump strokes.

16. The device according to any one of claims 10 to 15, **characterised in that** the control unit (19) is designed in such a way that the preset setpoint delivery rate of at least one of the pumps (10, 12, 16) is increased or reduced within a preset control range by a specific amount which is less than a preset limiting value, so that the difference between the weight reduction per unit of time of the sum of the weights of the first and third container (8, 14) and the weight increase per unit of time of the second container (13) corresponds to a preset value.

17. The device according to claim 16, **characterised in that** the control unit (19) is designed in such a way that the preset limiting value is a preset percentage of the preset setpoint delivery rate of the respective pump.

18. The device according to any one of claims 10 to 17, **characterised in that** the control unit (19) is designed in such a way that the deviation of the adopted delivery quantity of the respective pump (10, 12, 16) at the preset number of revolutions or pump strokes from the measured delivery quantity of the pump at the preset number of revolutions or pump strokes is compared with a preset limiting value, wherein an error signal is generated when the deviation is greater than the limiting value.

## Revendications

1. Procédé de commande d'un dispositif de traitement extracorporel du sang, qui présente :
une unité d'échange (1), qui est subdivisée par une membrane semi-perméable (2) en une première chambre (3) et une deuxième chambre (4), dans lequel la première chambre fait partie d'un circuit extracorporel du sang et la deuxième chambre fait partie d'un système de fluide,
une première pompe (10) pour le refoulement d'un fluide d'un premier contenant (8) avec un premier débit de refoulement dans la deuxième chambre de l'unité d'échange,
une deuxième pompe (12) pour le refoulement d'un fluide de la deuxième chambre de l'unité d'échange avec un deuxième débit de refoulement dans un deuxième contenant (13),
une troisième pompe (16) pour le refoulement d'un fluide avec un troisième débit de refoulement d'un troisième contenant (14) ou du premier contenant dans le circuit extracorporel du sang,
des premiers moyens (16) pour la détermination de la somme des poids du premier et troisième contenant ou du poids du premier contenant,
des deuxièmes moyens (18) pour la détermination du poids du deuxième contenant,
dans lequel les pompes (10, 12, 16) sont réglées en fonction de la somme des poids du premier et troisième contenant ou du poids du premier contenant d'une part et du poids du deuxième contenant d'autre part pour l'équilibrage des fluides,
**caractérisé en ce que**
la diminution de poids ou l'augmentation de poids d'au moins un des contenants (8, 13, 14) est mesurée dans l'intervalle de temps, dans lequel la pompe (10, 12, 16) attribuée au contenant respectif effectue un nombre prédéfini de rotations ou de courses de pompe,
la quantité de refoulement de la pompe respective (10, 12, 16) est déterminée pour le nombre prédéfini de rotations ou de courses de pompe à partir de la diminution de poids ou de l'augmentation de poids mesurée dans l'intervalle de temps déterminé, et
la quantité de refoulement de consigne de la pompe supposée lors de la commande de la pompe respective (10, 12, 16) pour le nombre prédéfini de rotations ou de courses de pompe est comparée avec la quantité de refoulement mesurée de la pompe pour le nombre prédéfini de rotations ou de courses de pompe, et
l'écart entre la quantité de refoulement de consigne supposée de la pompe respective pour le nombre prédéfini de rotations ou de courses de pompe et la quantité de refoulement mesurée de la pompe pour le nombre prédéfini de rotations ou de courses de pompe est à la base de la commande des pompes (10, 12, 16).

2. Procédé selon la revendication 1, **caractérisé en ce que** les pompes sont réglées pour l'équilibrage des fluides de telle sorte que la différence entre la diminution de poids par unité de temps correspond à la somme des poids du premier et troisième contenant (8, 14) et l'augmentation de poids par unité de temps du deuxième contenant (13) correspond à une valeur prédéfinie.

3. Procédé selon la revendication 1 ou 2, **caractérisé en ce que** la diminution de poids du premier contenant (8) est mesurée dans l'intervalle de temps, dans lequel la première pompe (10) effectue un nombre prédéfini de rotations ou de courses de pompe, et la quantité de refoulement de la première pompe est déterminée pour le nombre prédéfini de rotations ou de courses de pompe à partir de la diminution de poids mesurée,
l'augmentation de poids du deuxième contenant (13) est mesurée dans l'intervalle de temps, dans lequel la deuxième pompe (12) effectue un nombre prédéfini de rotations ou de courses de pompe, et la quantité de refoulement de la deuxième pompe est déterminée pour le nombre prédéfini de rotations ou de courses de pompe à partir de l'augmentation de poids mesurée,
la diminution de poids du troisième contenant (14) est mesurée dans l'intervalle de temps, dans lequel la troisième pompe (16) a effectué un nombre prédéfini de rotations ou de courses de pompe, et la quantité de refoulement de la troisième pompe est déterminée pour le nombre prédéfini de rotations ou de courses de pompe à partir de la diminution de poids mesurée, et
**que** les quantités de refoulement des pompes supposées lors de la commande des pompes (10, 12, 16) pour le nombre prédéfini de rotations ou de courses de pompe sont comparées avec les quantités de refoulement mesurées des pompes pour le nombre prédéfini de rotations ou de courses de pompe,
et l'écart entre les quantités de refoulement supposées des pompes pour le nombre prédéfini de rotations ou de courses de pompe et les quantités de refoulement mesurées des pompes pour le nombre prédéfini de rotations ou de courses de pompe est à la base de la commande des pompes.

4. Procédé selon l'une quelconque des revendications 1 à 3, **caractérisé en ce que** les premiers moyens (17) pour la détermination de la somme des poids du premier et troisième contenant comprennent une première balance pour peser simultanément le premier et troisième contenant (8, 14) et les deuxièmes moyens pour la détermination du poids du deuxième contenant comprennent une deuxième balance pour peser le deuxième contenant.

5. Procédé selon la revendication 4, **caractérisé en ce que** pour la détermination de la diminution de poids du premier contenant (8), la troisième pompe (16) est arrêtée dans l'intervalle de temps, dans lequel la première pompe effectue le nombre prédéfini de rotations ou de courses de pompe, ou le débit d'écoulement de la troisième pompe est réduit.

6. Procédé selon la revendication 4 ou 5, **caractérisé en ce que** pour la détermination de la diminution de poids du troisième contenant (14), la première pompe (10) est arrêtée dans l'intervalle de temps, dans lequel la troisième pompe effectue un nombre prédéfini de rotations ou de courses de pompe, ou le débit d'écoulement de la première pompe est réduit.

7. Procédé selon l'une quelconque des revendications 1 à 6, **caractérisé en ce que** le débit de refoulement de consigne prédéfini d'au moins une des pompes (10, 12, 16) est augmenté ou diminué à l'intérieur d'une plage de réglage prédéfinie d'une valeur déterminée, qui est inférieure à une valeur limite prédéfinie, de sorte que la différence entre la diminution de poids par unité de temps corresponde à la somme des poids du premier et troisième contenant (8, 14) et l'augmentation de poids par unité de temps du deuxième contenant (13) corresponde à une valeur prédéfinie.

8. Procédé selon la revendication 7, **caractérisé en ce que** la valeur limite prédéfinie est une valeur en pourcentage prédéfinie du débit de refoulement de consigne prédéfini de la pompe respective (10, 12, 16).

9. Procédé selon l'une quelconque des revendications 1 à 8, **caractérisé en ce que** l'écart entre la quantité de refoulement supposée de la pompe respective (10, 12, 16) pour le nombre prédéfini de rotations ou de courses de pompe et la quantité de refoulement mesurée de la pompe pour le nombre prédéfini de rotations ou de courses de pompe est comparé avec une valeur limite prédéfinie, dans lequel un signal d'erreur est généré, lorsque l'écart est supérieur à la valeur limite.

10. Dispositif de traitement du sang avec
une première pompe (10) pour le refoulement d'un fluide d'un premier contenant (8) avec un premier débit de refoulement dans la deuxième chambre (4) d'une unité d'échange (1), qui est subdivisée par une membrane semi-perméable (2) en une première chambre (3) et la deuxième chambre (4), dans lequel la première chambre (2) fait partie d'un circuit extracorporel du sang et la deuxième chambre (4) fait partie d'un système de fluide,
une deuxième pompe (12) pour le refoulement d'un fluide de la deuxième chambre (4) de l'unité d'échange (1) avec un deuxième débit de refoulement dans un deuxième contenant (13),
une troisième pompe (16) pour le refoulement d'un troisième fluide avec un troisième débit de refoulement d'un troisième contenant (14) ou du premier contenant (8) dans le circuit extracorporel du sang,
des premiers moyens (17) pour la détermination de la somme des poids du premier et troisième contenant (8, 14) ou du poids du premier contenant (8),
des deuxièmes moyens (18) pour la détermination du poids du deuxième contenant (13), et une unité de réglage (19) pour le réglage des débits de refoulement des pompes, qui est réalisée de telle sorte que les fluides sont équilibrés en fonction de la somme des poids du premier et troisième contenant (8, 14) ou du poids du premier contenant (8) d'une part et du poids du deuxième contenant (13) d'autre part,
**caractérisé en ce que** l'unité de réglage (19) est réalisée de telle sorte que la diminution de poids ou l'augmentation de poids d'au moins un des contenants (8, 13, 14) est mesurée dans l'intervalle de temps, dans lequel la pompe (10, 12, 16) attribuée au contenant respectif effectue un nombre prédéfini de rotations ou de courses de pompe,
la quantité de refoulement de la pompe respective (10, 12, 16) est déterminée pour le nombre prédéfini de rotations ou de courses de pompe à partir de la diminution de poids ou de l'augmentation de poids mesurée dans l'intervalle de temps, et
la quantité de refoulement de consigne de la pompe supposée lors de la commande de la pompe respective pour le nombre prédéfini de rotations ou de courses de pompe est comparée avec la quantité de refoulement mesurée de la pompe pour le nombre prédéfini de rotations ou de courses de pompe, et
l'écart entre la quantité de refoulement de consigne supposée de la pompe respective pour le nombre prédéfini de rotations ou de courses de pompe et la quantité de refoulement mesurée de la pompe pour le nombre prédéfini de rotations ou de courses de pompe est à la base de la commande des pompes (10, 12, 16).

11. Dispositif selon la revendication 10, **caractérisé en ce que** l'unité de réglage (19) est réalisée de telle sorte que pour l'équilibrage des fluides, les débits de refoulement des pompes (10, 12, 16) sont réglés de telle sorte que la différence entre la diminution de poids par unité de temps correspond à la somme des poids du premier et troisième contenant (8, 14) et l'augmentation de poids par unité de temps du deuxième contenant (13) correspond à une valeur prédéfinie.

12. Dispositif selon la revendication 10 ou 11, **caractérisé en ce que** l'unité de réglage (19) est réalisée de telle sorte que
la diminution de poids du premier contenant (8) est mesurée dans l'intervalle de temps, dans lequel la première pompe (10) effectue un nombre prédéfini de rotations ou de courses de pompe, et la quantité de refoulement de la première pompe (10) est déterminée pour le nombre prédéfini de rotations ou de courses de pompe à partir de la diminution de poids mesurée,
l'augmentation de poids du deuxième contenant (13) est mesurée dans l'intervalle de temps, dans lequel la deuxième pompe (12) effectue un nombre prédéfini de rotations ou de courses de pompe, et la quantité de refoulement de la deuxième pompe (12) est déterminée pour le nombre prédéfini de rotations ou de courses de pompe à partir de l'augmentation de poids mesurée,
la diminution de poids du troisième contenant (14) est mesurée dans l'intervalle de temps, dans lequel la troisième pompe (16) a effectué un nombre prédéfini de rotations ou de courses de pompe, et la quantité de refoulement de la troisième pompe (16) est déterminée pour le nombre prédéfini de rotations ou de courses de pompe à partir de la diminution de poids mesurée, et
**que** les quantités de refoulement des pompes supposées lors de la commande des pompes (10, 12, 16) pour le nombre prédéfini de rotations ou de courses de pompe sont comparées avec les quantités de refoulement mesurées des pompes pour le nombre prédéfini de rotations ou de courses de pompe,
et l'écart entre les quantités de refoulement supposées des pompes pour le nombre prédéfini de rotations ou de courses de pompe et les quantités de refoulement mesurées des pompes pour le nombre prédéfini de rotations ou de courses de pompe est à la base de la commande des pompes.

13. Dispositif selon l'une quelconque des revendications 10 à 12, **caractérisé en ce que** les premiers moyens (17) pour la détermination de la somme des poids du premier et troisième contenant (8, 14) comprennent une première balance (17) pour peser simultanément le premier et troisième contenant et les deuxièmes moyens pour la détermination du poids du deuxième contenant (13) comprennent une deuxième balance (18) pour peser le deuxième contenant.

14. Dispositif selon la revendication 13, **caractérisé en ce que** l'unité de réglage (19) est réalisée de telle sorte que pour la détermination de la diminution de poids du premier contenant (8), la troisième pompe (16) est arrêtée dans l'intervalle de temps, dans lequel la première pompe (10) effectue le nombre prédéfini de rotations ou de courses de pompe, ou le débit d'écoulement de la troisième pompe est réduit.

15. Dispositif selon la revendication 13 ou 14, **caractérisé en ce que** l'unité de réglage (19) est réalisée de telle sorte que pour la détermination de la diminution de poids du troisième contenant (14), la première pompe (10) est arrêtée dans l'intervalle de temps, dans lequel la troisième pompe (16) effectue un nombre prédéfini de rotations ou de courses de pompe, ou le débit d'écoulement de la première pompe est réduit.

16. Dispositif selon l'une quelconque des revendications 10 à 15, **caractérisé en ce que** l'unité de réglage (19) est réalisée de telle sorte que le débit de refoulement de consigne prédéfini d'au moins une des pompes (10, 12, 16) est augmenté ou diminué à l'intérieur d'une plage de réglage prédéfinie d'une valeur déterminée, qui est inférieure à une valeur limite prédéfinie, de sorte que la différence entre la diminution de poids par unité de temps corresponde à la somme des poids du premier et troisième contenant (8, 14) et l'augmentation de poids par unité de temps du deuxième contenant (13) corresponde à une valeur prédéfinie.

17. Dispositif selon la revendication 16, **caractérisé en ce que** l'unité de réglage (19) est réalisée de telle sorte que la valeur limite prédéfinie est une valeur en pourcentage prédéfinie du débit de refoulement de consigne prédéfini de la pompe respective.

18. Dispositif selon l'une quelconque des revendications 10 à 17, **caractérisé en ce que** l'unité de réglage (19) est réalisée de telle sorte que l'écart entre la quantité de refoulement supposée de la pompe respective (10, 12, 16) pour le nombre prédéfini de rotations ou de courses de pompe et la quantité de refoulement mesurée de la pompe pour le nombre prédéfini de rotations ou de courses de pompe est comparé avec une valeur limite prédéfinie, dans lequel un signal d'erreur est généré, lorsque l'écart est supérieur à la valeur limite.
